# EUROPÄISCHE PATENTSCHRIFT

(11) **EP 2 101 652 B1**
(45) Veröffentlichungstag und Bekanntmachung des Hinweises auf die Patenterteilung: **13.04.2016**
(21) Anmeldenummer: 07846533.3
(22) Anmeldetag: 06.11.2007
(51) Int. Cl.: A61B 17/04, A61B 17/11

(54) **AN EINE CHIRURGISCHE NÄHMASCHINE ANBAUBARE VORRICHTUNG ZUR BILDUNG EINER END-ZU-END-ANASTOMOSE AN ZWEI HOHLORGANEN**
DEVICE THAT CAN BE MOUNTED ON A SURGICAL SEWING MACHINE TO FORM AN END-TO-END ANASTOMOSIS BETWEEN TWO HOLLOW ORGANS
DISPOSITIF POUVANT ÊTRE MONTÉ SUR UNE MACHINE DE SUTURE CHIRURGICALE POUR FORMER UNE ANASTOMOSE TERMINO-TERMINALE ENTRE DEUX ORGANES CREUX

(30) Priorität: 09.11.2006 DE 102006053217
(43) Veröffentlichungstag der Anmeldung: 23.09.2009
(73) Patentinhaber: Karl Storz GmbH & Co. KG, 78532 Tuttlingen (DE)
(72) Erfinder: FRINGS, Hermann-Josef, 52072 Aachen (DE); MOLL, Clemens, 52064 Aachen (DE); MOLL, Philipp, 52076 Aachen (DE)
(86) Internationale Anmeldenummer: PCT/EP2007/009611
(87) Internationale Veröffentlichungsnummer: WO 2008/055655

(56) Entgegenhaltungen:
- EP-A- 1 415 597
- DE-A1- 10 116 171
- DE-A1- 10 240 331
- US-A- 4 553 961
- US-A- 4 778 467
- US-A1- 2003 045 891

## Beschreibung

Die Erfindung betrifft eine an eine chirurgische Nähmaschine mit mindestens einer motorisch angetriebenen Nadelstange mit einer fadenführenden Nadel, einem mit dieser zusammenwirkenden Greifer, einer Stichplatte und einem von einer Drückerstange aufgenommenen Niederhalter für das Nähgut anbaubare Vorrichtung zur Bildung einer End-zu- End-Anastomose an zwei Hohlorganen,

In der Medizintechnik ist es bei bestimmten Erkrankungen von Hohlorganen üblich, kranke Bereiche derselben, beispielsweise kranke Darmbereiche heraus zu schneiden und die beiden Schnittenden wieder miteinander zu verbinden. So wird beispielsweise das vom Magen kommende Darmende an den Anfang des weiterführenden Darmteiles angenäht.

Dies geschieht noch überwiegend von Hand, wobei eine mit einem Faden armierte Nadel in einem vom Arzt geführten Nadelhalter aufgenommen ist. Zur Bildung der Naht wird hierbei die erste Lefze mittels einer Pinzette festgehalten, die dann in unmittelbarer Nähe der Pinzette von der Nadel durchstochen wird. Mit der Pinzette wird dann die zweite Lefze aufgenommen und festgehalten, die dann ebenfalls im Bereich der Pinzette durchstochen wird. Dieser Vorgang wiederholt sich dann so oft, bis die Naht entlang des gesamten Umfanges der Darmteile gebildet ist, wobei darauf zu achten ist, daß einerseits die beiden Hohlorgane Stoß an Stoß zusammengenäht sind, und anderseits die innere Schleimhaut (Mycosa) nicht nach außen dringt.

Diese Art der Stichbildung entspricht im wesentlichen der Art der Bildung einer üblichen Handnaht einer Näherin oder einer Schneiderin, wobei die Qualität der Naht in erster Linie von der Aufmerksamkeit und insbesondere von der Geschicklichkeit der Nadelführung durch den Arzt abhängt.

Durch die DE 101 16 171 A1 ist eine als endoskopische Nähmaschine ausgebildete chirurgische Nähmaschine bekannt, die ein aus einem Gehäuseoberteil und einem sich an dieses anschließenden Gehäuseschaft gebildetes Gehäuse aufweist. Hierbei dient das Gehäuseoberteil in erster Linie zur Aufnahme von Antrieben für die Stichbildewerkzeuge, während der Gehäuseschaft zur Aufnahme von Mitteln zur Übertragung der von den Antrieben erzeugten Bewegungen auf die Stichbildewerkzeuge dient, die eine von einer Nadelstange aufgenommene fadenführende Nadel und einen mit dieser zusammenwirkenden Greifer aufweisen.

Im Bereich des unteren Endes des Gehäuseschaftes ist ferner sowohl eine Stichplatte, als auch ein von einem der im Gehäuseoberteil vorgesehenen Antriebe angetriebener und von einer Drückereinrichtung aufgenommener Niederhalter angeordnet.

Zur Bildung der Naht bewegt sich der Greifer nach dem Erfassen der durch die Nadel gebildeten Fadenschlinge entlang einer mehrdimensionalen Bewegungsbahn von einer unterhalb des Nähgutes gelegenen, die Fadenschlinge erfassenden Position in eine oberhalb des Nähgutes gelegene Position, in der das von der auf die Nähgutoberseite geführten Fadenschlinge gebildete Fadendreieck die Projektion der Nadelbahn umschließt.

Auf diese Weise ist es -wie in den Fig. 8 bis 12 der DE 101 16 171 A1 dargestellt- möglich, eine Überdecknaht unter Verwendung einer dem Stichtyp 501 entsprechenden Stichart zu bilden.

Damit ist diese endoskopische Nähmaschine zwar geeignet, flach aufeinander oder nebeneinander liegende Lefzen mittels einer Naht der vorbeschriebenen Art miteinander zu verbinden und damit die manuelle Nahtbildung zu ersetzen, jedoch ist diese endoskopische Nähmaschine zur Bildung von End-zu-End-Anastomosen an zwei Hohlorganen nicht vorgesehen und daher nicht optimal.

Der Erfindung liegt daher die Aufgabe zu Grunde, eine Vorrichtung zu schaffen, die einerseits die Bildung von End-zu-End-Anastomosen an zwei Hohlorganen ermöglicht, andererseits aber möglichst wenig in den Aufbau der vorbekannten chirurgischen Nähmaschine eingreift. Damit liegt der Erfindung die weitergehende Aufgabe zu Grunde, eine Vorrichtung zu schaffen, die ein "Rundum-Nähen" an den beiden zu verbindenden Hohlorganen in ihrem im wesentlichen zylinderförmigen Zustand ermöglicht, dabei aber gleichzeitig gewährleistet ist, daß die Innen- und Außenseiten der Hohlorgane nicht vertauscht werden.

Zur Lösung dieser Aufgabe dient eine an eine vorbeschriebene chirurgische Nähmaschine anbaubare Vorrichtung mit einem als Hohlzylinder ausgebildeten und einen Längsschlitz aufweisenden Träger für die miteinander zu verbindenden Randzonen der beiden Hohlorgane, der mittels einer Halterung mit dem Gehäuse der Nähmaschine derart verbindbar ist, daß seine Längsachse quer zur Bewegungsbahn der Nadel der Nähmaschine verläuft, und sein Auflagebereich für die beiden Hohlorgane im wesentlichen parallel zur Ebene der Auflagefläche der Stichplatte der Nähmaschine gerichtet ist. Weiterhin ist eine koaxial zum Träger angeordnete und relativ zu diesem drehbare Hülse vorgesehen, deren Mantelfläche als Auflagefläche für die Randbereiche der beiden Hohlorgane dient, wobei die Hülse ebenfalls einen sich zumindest über einen Teil ihrer Länge erstreckenden Längsschlitz aufweist. Um eine stets gleiche Stichlänge zu gewährleisten, ist die Hülse motorisch relativ zum Träger antreibbar, wobei die Bewegung des Antriebes der Hülse vom Antrieb der Nähmaschine derart abgeleitet ist, daß die Hülse in Abhängigkeit von der Bewegung der Nadel- oder der Drückerstange eine zur Nahtbildung phasengerechte intermittierende Drehbewegung ausführt, wodurch nicht nur eine stets gleiche Stichlänge erreicht wird, sondern auch sichergestellt ist, daß die Drehbewegung der Hülse in Bezug auf die Stichbildung stets phasengerecht zu dieser erfolgt.

Hierdurch bietet sich die Möglichkeit, das erste Hohlorgan in den Träger derart einzuführen, daß dessen Randbereich um einen bestimmten Betrag gegenüber dem Träger vorsteht und der vorstehende Randbereich derart über den Träger zurück gestülpt werden kann, daß die Außenseite des Hohlorganes an der Außenseite des Trägers an- bzw. aufliegt, wobei sich die Randzone des Hohlorganes unterhalb der Nadel befindet und auf der Stichplatte aufliegt Anschließend kann der Randbereich des zweiten Hohlorganes so weit auf den zurückgestülpten Bereich des ersten Hohlorgans aufgeschoben werden, bis sich seine Randzone randgleich mit der Randzone des ersten Hohlorganes ebenfalls unter der Nadel befindet und mit seiner Innenseite auf der Innenseite des ersten Hohlorganes aufliegt tn dieser Lage der beiden Hohlorgane kann mittels der chirurgischen Nähmaschine die die Randbereiche beider Hohlorgane verbindende Naht innerhalb der Randzonen beider Hohlorgane gebildet werden. Dabei sind die beiden Hohlorgane am Ende einer jeden Stichbildung jeweils um einen der Stichlänge der Naht entsprechenden Betrag unter der ausgestochenen Nadel weiter zu bewegen. Nach Beendigung der Naht können die beiden Hohlorgane, die während des Nähens ihre im wesentlichen zylindrische Form aufweisen, kurzzeitig etwas flach gedrückt werden, sodaß das erste Hohlorgan durch den im Träger vorgesehenen Längsschlitz aus dem Trägerherausbewegt und in Strecklage gebracht werden kann.

Da die Hülse unmittelbar auf dem Träger gelagert und somit um volle 360° drehbar ist, kann die herzustellende Naht innerhalb dieser 360° und somit innerhalb einer Umdrehung der Hülse hergestellt werden, wobei es lediglich erforderlich ist, darauf zu achten, daß einerseits die übereinander liegenden Randbereiche der beiden Hohlorgane stets ihre positionsgerechte Lage im Bereich der Stichbildestelle einnehmen und andererseits die Hülse nach jeder Stichbildung um den entsprechenden Wankelbetrag weiter gedreht wird.

Eine in konstruktiver Hinsicht einfache Lösung für die Ableitung der Drehbewegung der Hülse vom Antrieb der Nähmaschine ergibt sich dann, wenn die Hülse an einer ihrer Stirnseiten eine Zahnung aufweist, mit der eine am Träger gelagerte Antriebsklinke zusammenwirkt, die mittels eines verformbaren Übertragungsmittels mit dem Antrieb der Nadel- oder der Drückerstange antriebsmäßig verbindbar ist.

Dabei ist es zweckmäßig, wenn das Übertragungsmittel eine Kette oder ein Bowdenzug ist, dessen eines Endes mit dem Antrieb der Nadel- oder der Drückerstange verbindbar ist, und dessen anderes Ende an einem die Antriebsklinke aufnehmenden Hebel angreift, der am Träger schwenkbar gelagert ist, wobei die Antriebsklinke mittels einer Feder in Außereingriffstellung mit der Zahnung der Hülse gehalten ist.

Zur Erzielung der Ableitung der Bewegung der Hülse von der Schwenkbewegung der Antriebsklinke ist es vorteilhaft, wenn diese außermittig zur Hülse angeordnet ist und kraftschlüssig an deren Zahnung angreift, sodaß von den Schwenkbewegungen der Antriebsklinke eine phasengerechte intermittierende Drehbewegung der Hülse ableitbar ist.

Mit der erfindungsgemäßen Lösung ist angestrebt, nicht in den Aufbau und die Funktion der eingangs beschriebenen chirurgischen Nähmaschine einzugreifen und diese daher anbaubar an die chirurgische Nähmaschine zu gestalten, wobei gleichzeitig in der Nichtgebrauchslage eine möglichst raumsparende Form der Vorrichtung gewährleistet sein soll.

Dies wird in vorteilhafter Weise dadurch erreicht, daß die Halterung für den Träger von einem winkelförmigen Tragstück gebildet ist, dessen erster Arm über eine erste arretierbare Gelenkverbindung mit einem am Gehäuse der chirurgischen Nähmaschine befestigten Zwischenstück verbindbar ist, und dessen zweiter Arm mittels einer zweiten, ebenfalls arretierbaren Gelenkverbindung mit dem Träger verbindbar ist, und die Halterung von der die Wirkstellung des Trägers sichernden Lage in eine Lage bewegbar ist, in der der Träger im wesentlichen parallel zur Längsachse der Nähmaschine gerichtet ist.

Da der Abstand zwischen den Angriffspunkten des Bowdenzuges an der Nadel- oder Drückerstange und dem Hebel für die Antriebsklinke in der Wirkstellung des Trägers unterschiedlich zum Abstand der Angriffspunkte des Bowdenzuges in der unwirksamen Position des Trägers ist, ist der Bowdenzug über mehrere Umlenkstellen geführt, wovon mindestens eine am Träger und eine weitere am ersten Arm der Halterung oder am Gehäuseschaft der chirurgischen Nähmaschine angeordnet ist.

Weitere Vorteile und Einzelheiten der Erfindung ergeben sich anhand der nachfolgenden Beschreibung eines in der beigefügten Zeichnung dargestellten Ausführungsbeispiels der Erfindung.

Es zeigt:
- **Fig. 1:**: die Ausgangslage der beiden miteinander zu verbindenden Hohlorgane A,B;
- **Fig. 2:**: eine schematische Darstellung des unteren Bereiches einer chirurgischen Nähmaschine gemäß DE 101 16 171 A1 mit den für den Nähvorgang vorbereiteten Hohlorganen;
- **Fig. 3:**: den unteren Bereich der Nähmaschine mit einer daran angebauten erfindungsgemäßen Vorrichtung mit in Nählage befindlichen Hohlorganen;
- **Fig. 4:**: eine schaubildliche Darstellung der erfindungsgemäßen Vorrichtung während des Nähvorganges;
- **Fig. 5:**: eine schaubildliche Darstellung der erfindungsgemäßen Vorrichtung nach dem Nähvorgang während des Entnehmens der Hohlorgane A, B;
- **Fig. 6:**: eine Draufsicht auf die durch eine Rundum-Naht miteinander verbundenen Hohlorgane A,B in Schnittdarstellung;
- **Fig. 7:**: einen Längsschnitt des Trägers mit dessen Halterung und dem Gehäuseschaft der Nähmaschine;
- **Fig. 8:**: einen Teilschnitt des Trägers und der Hülse mit deren Antrieb und dem Gehäuseschaft der Nähmaschine;
- **Fig. 9:**: eine Vorderansicht der sich in der Einführstellung befindlichen erfindungsgemäßen Vorrichtung mit dem Gehäuseschaft der Nähmaschine;
- **Fig.**: **10:** eine vergrößerte Darstellung der verrasteten Stellung des ersten Armes des Tragstückes mit dem Gehäuseschaft der Nähmaschine;

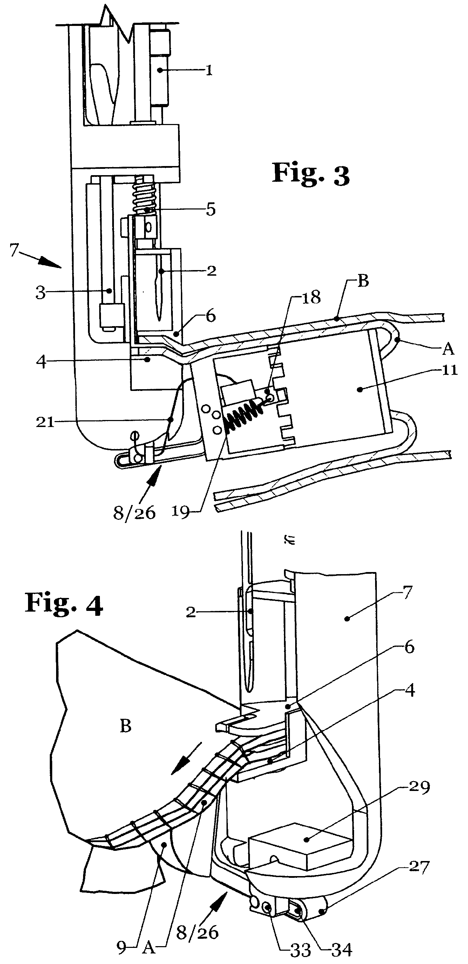

In Fig. 3 ist der untere Bereich einer chirurgischen Nähmaschine gezeigt, wie sie beispielsweise in der DE 101 16 171 A1 bzw der EP 1 372 490 B1 dargestellt und beschrieben ist.

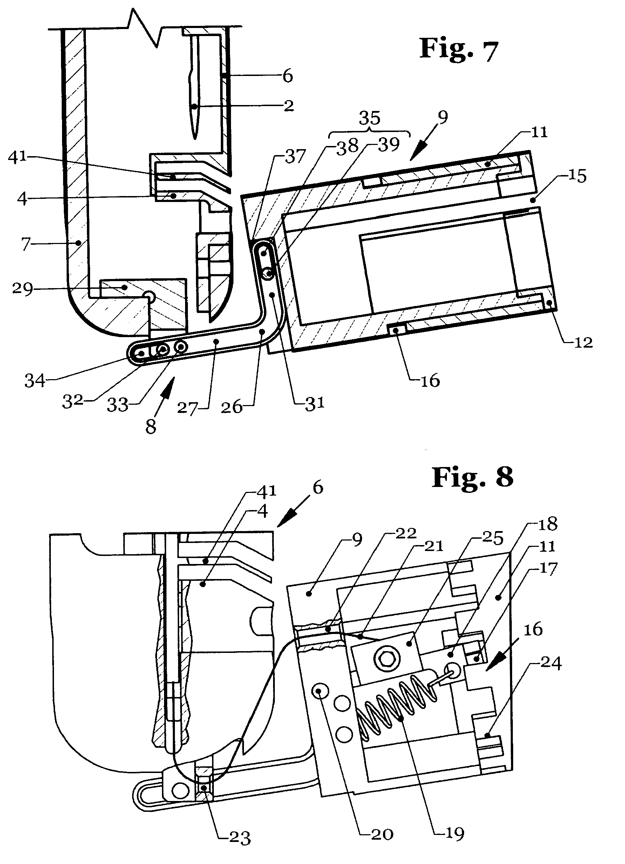

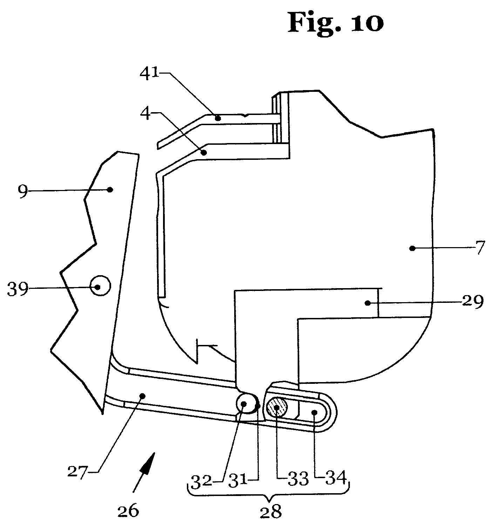

Diese Nähmaschine weist eine motorisch bewegte Nadelstange 1 mit einer fadenführenden Nadel 2 auf, die mit einem Greifer zur Bildung einer Kettenstichnaht des Stichtyps 501 oder eines hierzu ähnlichen Stichtypes zusammenwirkt. Die hier im Detail nicht weiter interessierende Stichbildung ist in den Fig. 8 bis 12 der vorgenannten Veröffentlichungen dargestellt und auch in der Beschreibung im Detail erläutert.

Neben der Nadel 2 und dem an einer Greiferstange 3 befestigten -nicht dargestellten- Greifer weist die Nähmaschine eine Stichplatte 4 mit einem schlitzartigen Stichloch und einen von einer Drückerstange 5 aufgenommenen, ebenfalls mit einem Schlitz versehenen Niederhalter 6 für die Hohlorgane A,B auf, der in Abhängigkeit von der Bewegung der Nadel 2 zur Erleichterung der Vorschubbewegung der beiden Hohlorgane A,B während den ausgestochenen Phasen der Nadel 2 periodisch angehoben wird.

Beim gezeigten Ausführungsbeispiel der Erfindung ist im unteren Bereich des Gehäuseschaftes 7 der chirurgischen Nähmaschine gemäß der DE 101 16 171 A1 bzw der EP 1 372 490 B1 eine mit diesem lösbar verbindbare Halterung 8 für einen als Hohlzylinder ausgebildeten Träger 9 vorgesehen, auf dem eine Hülse 11 drehbar gelagert ist, wobei an einer der Stirnseiten der Hülse 11 ein Klemmring 12 angeordnet ist.

Die Mantelfläche der Hülse 11 dient als Auflagefläche für die miteinander zu verbindenden Randbereiche der beiden Hohlorgane A,B. Der Träger 9 und die Hülse 11 sind in Bezug auf die Nähmaschine derart angeordnet, daß ihre Längsachsen quer zur Bewegungsbahn der Nadel 2 der Nähmaschine verlaufen. Ferner ist die Anordnung von Träger 9 und Hülse 11 derart getroffen, daß der Auflagebereich der Hülse für die beiden Hohlorgane A,B im wesentlichen parallel, vorzugsweise aber im spitzen Winkel zur Ebene der Auflagefläche der Stichplatte 4 der Nähmaschine gerichtet ist. Der Träger 9 und die Hülse 11 weisen zu einem noch später zu erläuternden Zweck je einen Längsschlitz 13 bzw 14 auf, der sich über mindestens einen Teil der Länge des Trägers 9 bzw der Hülse 11 bzw erstreckt. Dabei kann die Relativlage zwischen dem Träger 9 und der Hülse 11 durch Drehen der Hülse 11 derart eingestellt werden, daß die beiden Längsschlitze 13,14 zueinander deckungsgleich verlaufen. Auch der Klemmring 12 ist mit einem entsprechenden Längsschlitz 15 versehen, der dem in der Hülse 11 vorgesehenen Längsschlitz 14 entspricht. Der Klemmring 12 wird daher derart in die Hülse 11 eingesetzt, daß die beiden Längsschlitze 14,15 deckungsgleich verlaufen.

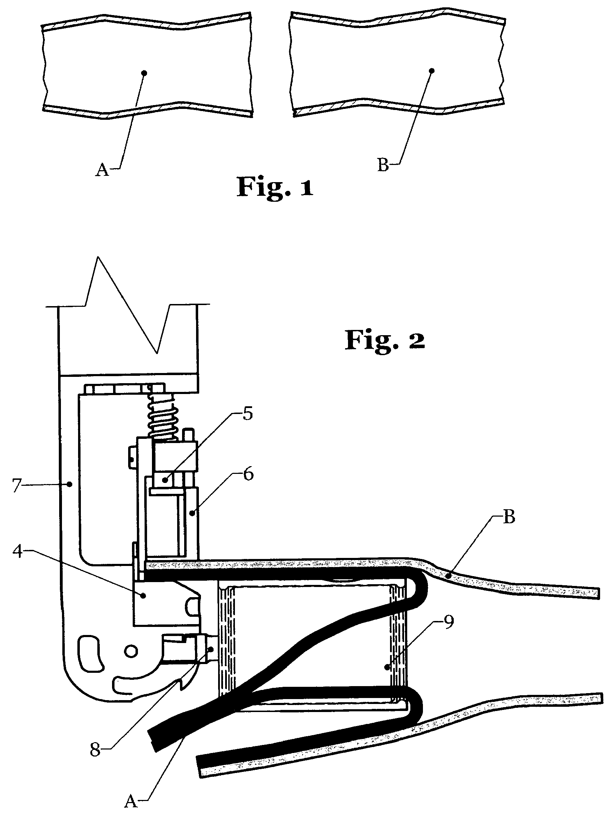

In Fig. 1 sind die Randbereiche zweier eine im wesentlichen zylindrische Form aufweisenden Hohlorgane A,B gezeigt, die an ihren einander benachbarten Randzonen mittels einer Naht verbunden werden sollen. Hiervon ausgehend wird das freie Ende des Hohlorganes A derart in den Träger 9 bzw die Hülse 11 eingeführt, daß sein Randbereich um einen bestimmten Betrag gegenüber dem Träger 9 bzw der Hülse 11 vorsteht, sodaß der vorstehende Randbereich derart über die Hülse 11 zurück gestülpt werden kann, daß die Außenseite des Hohlorganes A auf der Außenseite der Hülse an- bzw. aufliegt, wobei sich die Randzone des Randbereiches des Hohlorganes A unterhalb der Nadel 2 der chirurgischen Nähmaschine befindet und auf deren Stichplatte 4 aufliegt. Anschließend kann der Randbereich des Hohlorganes B so weit auf den zurück gestülpten Bereich des Hohlorgans A aufgeschoben werden, bis sich auch seine Randzone deckungsgleich mit der Randzone des Hohlorganes A unter der Nadel 2 befindet und mit seiner Innenseite auf der nach außen gestülpten Innenseite des Hohlorganes A aufliegt.

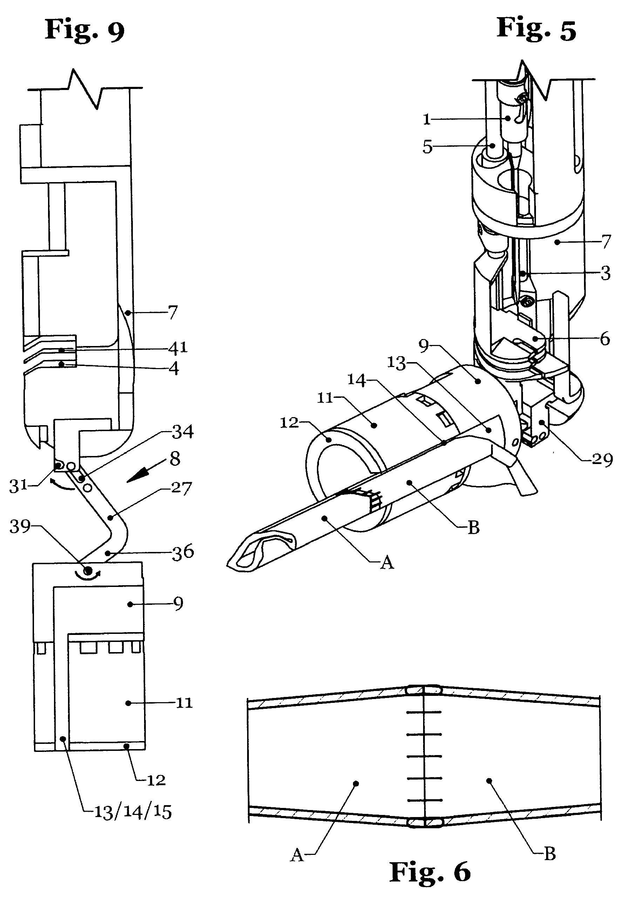

In dieser Lage der beiden Hohlorgane A,B kann mittels der chirurgischen Nähmaschine innerhalb der Randzonen beider Randbereiche der Hohlorgane A,B eine diese miteinander verbindende Naht gebildet werden. Die Naht kann beispielsweise eine einfädige Überdecknaht der Stichtype 501 sein, deren Bildung in den Fig. 8 bis 12 der DE 101 16 171 A1 oder der EP 1 372 490 B1 dargestellt und beschrieben ist. Bei dieser Stichtype wird die an der Unterseite des Nähgutes gebildete Fadenschleife durch den Greifer von der Unterseite des Nähgutes zu dessen Oberseite geführt und dort fortlaufend durch die in die Fadenschleife einstechende Nadel fixiert. Diese Stichtype ist, da sich die übereinander liegend verbundenen Wandungen beider Hohlorgane sehr leicht in eine in Fig. 6 gezeigte Strecklage bringen lassen, zum vorliegenden Zweck besonders gut geeignet.

Zur fortlaufenden Stichbildung und damit zur Bildung der Rundum-Naht müssen die beiden Hohlorgane A,B am Ende einer jeden Stichbildung durch aufeinander folgendes Drehen der Hülse 11 jeweils um einen der Stichlänge der Naht entsprechenden Betrag bei ausgestochener Nadel 2 so lange phasengerecht zur Stichbildung weiter bewegt werden, bis der Nahtanfang erreicht und damit die Rundum-Naht gebildet ist. Nach Beendigung der Naht werden die beiden durch die Naht miteinander verbundenen Hohlorgane A,B, die während des Nähens ihre im wesentlichen zylindrische Form aufweisen, zunächst so weit von der Stichbildestelle der chirurgischen Nähmaschine wegbewegt, bis sich ihre mit der Naht versehenen Randbereiche im Bereich der Längsschlitze 13,14 von Träger 9 und Hülse 11 befinden. Nachdem die Längsschlitze 13, 14 durch Drehen der Hülse 11 in eine deckungsgleiche Lage gebracht sind, werden die Hohlorgane A,B kurzzeitig etwas flach gedrückt, sodaß das Hohlorgan A durch die im Träger 9 bzw der Hülse vorgesehenen Längsschlitze 13, 14 aus der Hülse 11 bzw dem Träger 9 herausbewegbar ist und beide Hohlorgane A,B in Strecklage gebracht werden können, in der sie die in Fig. 6 gezeigte Lage einnehmen.

In einer bevorzugten Ausführungsform ist die erfindungsgemäße Vorrichtung mit einem Drehantrieb für die Hülse 11 versehen, dessen Antriebsbewegung von der Hubbewegung der Nadelstange 2 oder der Drückerstange 5 des Niederhalters 6 der chirurgischen Nähmaschine abgenommen ist, sodaß die Hülse 11 eine zur Stichbildung phasengerechte intermittierende Drehbewegung ausführt.

Hierzu ist an der dem Klemmring 12 abgekehrten Stirnseite der Hülse 11 (Fig. 8) eine Zahnung 16 ausgebildet, mit der eine Antriebsklinke 17 zusammenwirkt, die an einem am Träger 9 mittels eines Zapfens 20 schwenkbar gelagerten Hebel 18 ausgebildet ist. Die Antriebsklinke 17 wird mittels einer einerends am Hebel 18 und anderenends am Träger 9 angreifenden Feder 19 außer Eingriff mit der Zahnung 16 der Hülse 11 gehalten. Am Hebel 18 greift ein beim Ausführungsbeispiel als Bowdenzug 21 ausgebildetes verformbares Übertragungsmittel an, dessen anderes Ende an der Drückerstange 5 für den Niederhalter 6 angelenkt ist. Hierzu ist der Bowdenzug sowohl über eine am Träger 9 befestigte erste Führung 22, als auch und über eine am Gehäuseschaft 7 der chirurgischen Nähmaschine angeordnete zweite Führung 23 geführt. Damit wird die Antriebsklinke 17 während der Hubbewegung des Niederhalters 6 gleichzeitig mit diesem angehoben.

Die Antriebsklinke 17 ist außermittig zur Hülse 11 angeordnet und liegt während ihrer durch den Bowdenzug 21 eingeleiteten Schwenkbewegung jeweils an der Unterseite eines Zahnes der Zahnung 16 der Hülse 11 kraftschlüssig an. Hierdurch wird von ihrer Schwenk- bzw Hubbewegung eine im wesentlichen tangential gerichtete Kraftkomponente abgeleitet, die eine Drehbewegung der Hülse 11 bewirkt, die hierdurch phasengerecht zur Hubbewegung der Drückerstange 5 des Niederhalters 6 erfolgt. Da die Antriebsklinke 17 an der Unterseite der Zahnung 16 kraftschlüssig anliegt, und der jeweilige Zahn sich während der Drehbewegung der Hülse 11 über die Antriebsklinke 17 hinweg bewegt, kommt die Antriebsklinke 17 ohne zusätzliche Steuermittel nach einer der Breite des jeweiligen Zahnes entsprechenden Drehbewegung der Hülse 11 außer Eingriff und wird durch die Feder 19 in ihre Ruhestellung zurückgeführt. Um während der eingestochenen Phase der Nadel einer ungewollten Bewegung der Hülse entgegen zu wirken, ist am Träger 9 eine als Biegefeder ausgebildete Bremse 24 vorgesehen, die an der Innenseite der Zahnung 16 anliegt.

In einer bevorzugten Ausführungsform greift der Bowdenzug 21 nicht unmittelbar am Hebel 18, sondern an einem auf diesem verschieb- und feststellbar angeordneten Schiebestück 25 an, sodaß die Größe der Schwenkbewegung der Antriebsklinke 18 bzw deren Anpreßkraft an die Zahnung 16 innerhalb bestimmter Grenzen einstellbar ist, sodaß auch die Größe der jeweiligen Drehbewegung der Hülse 11 und damit die Stichlänge der zu bildenden Naht innerhalb entsprechender Grenzen einstellbar ist.

Zur Verbindung des Trägers 9 mit dem Gehäuseschaft 7 der chirurgischen Nähmaschine umfaßt die Halterung 8 ein winkelförmiges Tragstück 26 (Fig. 7), dessen erster Arm 27 mittels einer ersten arretierbaren Gelenkverbindung 28 mit einem am Gehäuseschaft 7 der chirurgischen Nähmaschine befestigten Zwischenstück 29 verbindbar ist, dessen zweiter Arm 31 mittels einer zweiten, ebenfalls arretierbaren Gelenkverbindung 32 mit dem Träger 9 verbindbar ist.

Dabei umfaßt die erste Gelenkverbindung 28 zwischen dem ersten Arm 27 des Tragstückes 26 und dem Zwischenstück 29 eine an diesem vorgesehene einseitig offene Rastöffnung 31 in die ein im Arm 27 des Tragstückes 26 angeordneter Stift 32 ragt und in dieser verrastbar ist. Am Zwischenstück 26 ist ein zum Stift 32 parallel verlaufender Lagerzapfen 33 befestigt, der in ein im Arm 27 der Halterung vorgesehenes Langloch 34 eingreift. Diese Relativlage des Armes 27 des winkelförmiges Tragstückes 26 bzw der Halterung 8, die durch den in der Rastöffnung 31 verrasteten Stift 32 und den in das Langloch 34 des Armes 27 ragenden Lagerzapfen 33 gesichert ist, entspricht der Wirkstellung des Trägers 9 bzw der Hülse 11.

Zum Verschwenken der Halterung 8 wird deren Arm 27 zunächst so weit vom Zwischenstück 29 wegbewegt, daß der Stift 32 außer Eingriff von der Rastöffnung 31 kommt. Während dieser Bewegung gleitet der Lagerzapfen 33 zum anderen Ende des Langloches 34 und bildet in dieser Lage eine Schwenkachse für den Arm 27 bzw das Tragstück 26, sodaß dieses eine Schwenkbewegung von ca 90° um den Lagerzapfen 33 ausführt.

Zur Bildung der Gelenkverbindung 35 zwischen dem zweiten Arm 36 des Tragstückes 26 und dem Träger 9 ist an dessen Stirnseite eine der Form des zweiten Armes 36 entsprechende Ausnehmung 37 vorgesehen, in die der zweite Arm 36 eingreift. Dabei ist im Bereich des freien Endes des Armes 36 ein Langloch 38 eingearbeitet, in das ein im Träger 9 befestigter Lagerzapfen 39 eingreift. In der Wirkstellung des Trägers 9 befindet sich der Lagerzapfen 39 im Bereich des -bezogen auf Fig. 7- unteren Ende des Langloches 38 und bewegt sich während der um den Lagerzapfen 33 erfolgenden Schwenkbewegung des Tragstückes 26 zum oberen Ende des Langloches 38 hin. In dieser Lage des Lagerzapfens 39 dient dieser als Schwenkachse für den Träger 9, sodaß dieser in eine im wesentlichen mit der Längsachse der Nähmaschine fluchtende Lage geschwenkt werden kann, die in Fig. 9 dargestellt ist.

In einer bevorzugten Weiterbildung der Ausführungsform der Vorrichtung ist oberhalb der Stichplatte 4 eine zu dieser im wesentlichen parallel verlaufende Zwischenplatte 41 vorgesehen, die in ihrem vorderen Bereich leicht abgeschrägt ist. Hierdurch bietet sich die Möglichkeit, die Außenseite des Randbereiches des Hohlorganes A auf die Stichplatte 4 und die Innenseite des Randbereiches des Hohlorganes B auf die Zwischenplatte 41 aufzulegen, sodaß sicher gestellt ist, daß die beiden Innenseiten der beiden Hohlorgane A,B im Bereich des Niederhalters 6 nicht unmittelbar aufeinander liegen. Hierdurch wird ausgeschlossen, daß somit die Innenseiten der beiden Hohlorgane A,B durch den Druck des Niederhalters 6 unmittelbar gegeneinander gedrückt werden.

Hierbei kann die Zwischenplatte 41 mittels einer am Niederhalter 6 vorgesehenen -nicht dargestellten- Führung parallel zu diesem geführt sein, und zur Erleichterung des Einführens der Randbereiche der beiden Hohlorgane A,B in der angehobenen Stellung des Niederhalters 6 in höhenmäßigen Abstand von der Stichplatte 4 gehalten sein.

In einer weiteren Ausführungsform der Erfindung kann anstelle des zylinderförmigen Trägers mit koaxial zu diesem angeordneter Hülse auch ein im wesentlichen stab- oder halbschalenförmig ausgebildeter Träger verwendet werden, an dessen beiden Längsseiten je ein eben oder bogenförmig ausgestaltetes Auflageteil angelenkt ist, wobei die jeweilige Auflagefläche des bogenförmig ausgebildeten Auflageteiles konkav oder konvex geformt sein kann.

Dabei kann der stab- oder halbschalenförmig ausgebildete Träger derart an den zweiten Arm des Tragstückes angelenkt sein, daß von der ihn von der Ruhestellung in seine Wirkstellung bzw von seiner Wirkstellung in seine Ruhestellung führenden Schwenkbewegung jeweils eine entsprechende Bewegung der jeweils beiden Auflageteile derart ableitbar ist, daß diese während der Schwenkbewegung des Trägers von der Ruhestellung in seine Wirkstellung in ihre ausgebreitete Lage während der ihn von seiner Wirkstellung in seine Ruhestellung führenden Schwenkbewegung von ihrer ausgebreiteten Wirkstellung in eine zusammengeklappte Lage überführt werden, in der sie im wesentlichen mit der Längsachse der chirurgischen Nähmaschine fluchten.

Diese Ausbildung ist insbesondere dann von Vorteil, wenn an größeren Organen eine Naht gebildet werden soll und die zu verbindenden Organe während der Nahtbildung in einer bestimmten Relativlage zu den zu vernähenden Bereichen gehalten werden sollen.

Im chirurgischen Bereich sind neben der Verbindungstechnik "Nähen mittels Faden" noch weitere Verbindungstechniken wie beispielsweise "Klammern" bekannt, die sowohl in der laparoskopischen als auch der offenen Chirurgie zum Einsatz kommen.

Vorstehend wurden die erfindungsgemäße Vorrichtung und das dieser zugrunde liegende Verfahren zwar am Beispiel des Nähens erläutert, jedoch ist die Anwendung der erfindungsgemäßen Lehre nicht auf das Nähen beschränkt. Vielmehr kann diese im Hinblick darauf, daß sowohl beim Nähen als auch beim Klammern, das eigentliche Verbindungsmittel (Faden bzw Klammern) bei beiden Techniken von der einen Seite der Lefzen zu deren anderen Seite geführt werden, wobei beim Nähen die Sicherung der Naht durch wiederholtes Verschlingen des Fadens erfolgt, während beim Klammern der feste Sitz der Klammern durch Umbiegen der freien Enden der Klammern erreicht wird, ohne weiteres auch beim Klammern eingesetzt werden.

Ebenso ist es möglich, die Verbindung der beiden Lefzen unter Nutzung der erfindungsgemäßen Lehre durch Laser-Schweißung unter Zuhilfenahme entsprechender Schweißhilfsmittel herzustellen.

## Patentansprüche

1. An eine chirurgische Nähmaschine mit mindestens einer motorisch angetriebenen Nadelstange mit einer fadenführenden Nadel, einem mit dieser zusammenwirkenden Greifer, einer Stichplatte und einem von einer Drückerstange aufgenommenen Niederhalter für das Nähgut anbaubare Vorrichtung zur Bildung einer End-zu- End-Anastomose an zwei Hohlorganen,
**gekennzeichnet durch**
einen als Hohlzylinder ausgebildeten und einen Längsschlitz (13) aufweisenden Träger (9) für die miteinander zu verbindenden Randbereiche der beiden Hohlorgane, der mittels einer Halterung (8) mit dem Gehäuseschaft (7) der Nähmaschine derart verbindbar ist, daß seine Längsachse quer zur Bewegungsbahn der Nadel (2) der Nähmaschine verläuft, und sein Auflagebereich für die beiden Hohlorgane im wesentlichen parallel zur Ebene der Auflagefläche der Stichplatte (4) der Nähmaschine gerichtet ist, wobei eine koaxial zum Träger (9) angeordnete und relativ zu diesem drehbare Hülse (11), deren Mantelfläche als Auflagefläche für die Endbereiche der beiden Hohlorgane dient, einen sich über zumindest einen Teil ihrer Länge erstreckenden Längsschlitz (14) aufweist
und
daß die Hülse (11) mittels eines Antriebes relativ zum Träger (9) drehbar ist, und die Bewegung des Antriebes der Hülse (11) vom Antrieb der Nähmaschine derart abgeleitet ist, daß die Hülse (11) in Abhängigkeit von der Bewegung der Nadelstange (1) phasengerecht zur Nahtbildung eine intermittierende Drehbewegung ausführt.

2. An eine chirurgische Nähmaschine anbaubare Vorrichtung nach Anspruch 1 **dadurch gekennzeichnet, daß** die Hülse (11) an einer ihrer Stirnseiten mittels eines Klemmringes (12) verschließbar ist und der Klemmring (12) einen dem Längsschlitz (14) der Hülse (11) entsprechenden Längsschlitz (15) aufweist.

3. An eine chirurgische Nähmaschine anbaubare Vorrichtung nach Anspruch 2, **dadurch gekennzeichnet, daß** die Hülse (11) an ihrem dem Klemmring (12) abgekehrten Ende eine Zahnung (16) aufweist, mit der eine am Träger (9) gelagerte Antriebsklinke (17) zusammenwirkt, die mittels eines verformbaren Übertragungsmittels (21) mit dem Antrieb der Nadelstange (1) antriebsmäßig verbindbar ist.

4. An eine chirurgische Nähmaschine anbaubare Vorrichtung nach Anspruch 1, **dadurch gekennzeichnet, daß** die Halterung (8) für den Träger (9) von einem winkelförmigen Tragstück (26) gebildet ist, dessen erster Arm (27) über eine erste Gelenkverbindung (28) mit einem am Gehäuse der chirurgischen Nähmaschine befestigten Zwischenstück (29) verbindbar ist, und dessen zweiter Arm (36) mittels einer zweiten Gelenkverbindung (35) mit dem Träger (9) verbindbar ist, und die Halterung (8) von der die Wirkstellung des Trägers (9) sichernden Lage in eine Lage bewegbar ist, in der der Träger (9) im wesentlichen parallel zur Längsachse der Nähmaschine gerichtet ist.

5. An eine chirurgische Nähmaschine anbaubare Vorrichtung nach Anspruch 4, **dadurch gekennzeichnet, daß** die beiden Gelenkverbindungen (28, 35) arretierbar sind.

6. Chirurgische Nähmaschine mit mindestens einer motorisch angetriebenen Nadelstange mit einer fadenführenden Nadel, einem mit dieser zusammenwirkenden Greifer, einer Stichplatte und einem von einer Drückerstange aufgenommenen Niederhalter für das Nähgut, **gekennzeichnet durch** eine Vorrichtung zur Bildung einer End-zu-End-Anastomose an zwei Hohlorganen, aufweisend
a.) einen als Hohlzylinder ausgebildeten Träger (9) mit einer koaxial zu diesem angeordneten Hülse (11), wobei die Längsachse des Trägers (9) quer zur Bewegungsbahn der Nadel (2) verläuft, und die Mantelfläche der Hülse (11) parallel oder in spitzem Winkel zur Ebene der Stichplatte (4) gerichtet ist und als Auflagefläche für die Randbereiche der beiden zu verbindenden Hohlorgane dient, wobei
b.) der Träger (9) und die Hülse (11) je einen sich über zumindest einen Teil ihrer Länge erstreckenden Längsschlitz (13, 14) aufweisen,
c.) die Hülse (11) mittels eines Antriebes relativ zum Träger (9) drehbar ist, und der Antrieb der Hülse (11) vom Antrieb der Nähmaschine derart abgeleitet ist, daß die Hülse (11) in Abhängigkeit von der Bewegung der Nadelstange (1) eine zur Stichbildung phasengerechte intermittierende Drehbewegung ausführt.

7. Chirurgische Nähmaschine nach Anspruch 6, **dadurch gekennzeichnet, daß** die Hülse (11) an einer ihrer Stirnseiten eine Zahnung (16) aufweist, mit der eine am Träger (9) gelagerte Antriebsklinke (17) zusammenwirkt, die mittels eines verformbaren Übertragungsmittels (21) mit dem Antrieb der Nadelstange (1) antriebsmäßig verbunden ist.

8. Chirurgische Nähmaschine nach Anspruch 7, **dadurch gekennzeichnet, daß** das Übertragungsmittel ein Bowdenzug (21) ist, dessen eines Endes mit dem Antrieb der Nadelstange (1) verbunden ist, und dessen anderes Ende an einem die Antriebsklinke (17) aufnehmenden Hebel (18) angreift, der am Träger (9) schwenkbar gelagert ist.

9. Chirurgische Nähmaschine nach Anspruch 7, **dadurch gekennzeichnet, daß** die Antriebsklinke (17) außermittig zur Hülse (11) angeordnet ist und kraftschlüssig an der Innenseite der Zahnung (16) angreift.

10. Chirurgische Nähmaschine nach Anspruch 6 **dadurch gekennzeichnet dass** die Halterung (8) für den Träger (9) von einem winkelförmigen Tragstück (26) gebildet ist, dessen erster Arm (27) mit dem Gehäuse der Nähmaschine verbindbar ist, und dessen zweiter Arm (36) mit dem Träger (9) verbindbar ist, wobei die Halterung (8) von der die Wirkstellung des Trägers (9) sichernden Lage in eine Lage bewegbar ist, in der der Träger im wesentlichen parallel zur Längsachse der Nähmaschine gerichtet ist.

11. Chirurgische Nähmaschine nach Anspruch 10, **dadurch gekennzeichnet, dass** der erste Arm (27) des Tragstückes (26) und die Nähmaschine über eine erste Gelenkverbindung (28) miteinander verbindbar sind, und der zweite Arm (36) des Tragstückes (26) und der Träger (9) über eine zweite Gelenkverbindung (35) miteinander verbindbar sind.

12. Chirurgische Nähmaschine nach Anspruch 11, **dadurch gekennzeichnet, dass** die erste Gelenkverbindung (28) lösbar mit dem Gehäuseschaft (7) der Nähmaschine verbunden ist.

13. Chirurgische Nähmaschine nach Anspruch 7, **dadurch gekennzeichnet, daß** der Bowdenzug (21) über mehrere Umlenkstellen (22, 23) geführt ist, wovon mindestens eine (22) am Träger (9) und eine weitere am ersten Arm (27) des Tragstückes (26) oder am Gehäuseschaft (7) angeordnet ist

14. Chirurgische Nähmaschine nach Anspruch 6, **dadurch gekennzeichnet, daß** die Stichplatte (4) eine Auflagefläche für das erste Hohlorgan aufweist und in vertikalem Abstand hierzu eine Zwischenplatte (41) vorgesehen ist, die eine Auflagefläche für das zweite Hohlorgan aufweist.

## Claims

1. Device for forming an end-to-end anastomosis on two hollow organs, which device is attachable to a surgical suturing machine having at least one motor-driven needle bar with a thread-carrying needle, a shuttle cooperating therewith, a needle plate, and a holding-down mechanism received by a push rod and provided for the suture material,
**characterized by**
a carrier (9), designed as a hollow cylinder and having a longitudinal slot (13), for the edge areas of the two hollow organs to be connected to each other, which carrier (9) is connectable, by means of a bracket (8), to the housing shaft (7) of the suturing machine in such a way that its longitudinal axis runs transversely with respect to the path of movement of the needle (2) of the suturing machine, and its contact area for the two hollow organs is directed substantially parallel to the plane of the contact surface of the needle plate (4) of the suturing machine, wherein a sleeve (11), which is arranged coaxially with respect to the carrier (9) and is rotatable relative to the latter and of which the jacket surface serves as a contact surface for the end areas of the two hollow organs, has a longitudinal slot (14) extending along at least part of its length,
and
in that the sleeve (11) is rotatable relative to the carrier (9) by means of a drive, and the movement of the drive of the sleeve (11) is diverted from the drive of the suturing machine in such a way that the sleeve (11) executes an intermittent rotary movement in accordance with the movement of the needle bar (1) and in phase with the suture formation.

2. Device according to Claim 1 and attachable to a surgical suturing machine, **characterized in that** the sleeve (11) can be closed at one of its ends by means of a clamping ring (12), and the clamping ring (12) has a longitudinal slot (15) corresponding to the longitudinal slot (14) of the sleeve (11).

3. Device according to Claim 2 and attachable to a surgical suturing machine, **characterized in that** the sleeve (11), at its end facing away from the clamping ring (12), has a toothing (16) with which a driving pawl (17) mounted on the carrier (9) cooperates, said driving pawl (17) being connectable in drive terms to the drive of the needle bar (1) by a deformable transmission means (21).

4. Device according to Claim 1 and attachable to a surgical suturing machine, **characterized in that** the bracket (8) for the carrier (9) is formed by an angular carrying piece (26) whose first arm (27) is connectable via a first hinge connection (28) to a spacer (29) secured on the housing of the surgical suturing machine, and whose second arm (36) is connectable to the carrier (9) by means of a second hinge connection (35), and the bracket (8) is movable, from the position securing the operating state of the carrier (9), to a position in which the carrier (9) is directed substantially parallel to the longitudinal axis of the suturing machine.

5. Device according to Claim 4 and attachable to a surgical suturing machine, **characterized in that** the two hinge connections (28, 35) are lockable.

6. Surgical suturing machine having at least one motor-driven needle bar with a thread-carrying needle, a shuttle cooperating therewith, a needle plate, and a holding-down mechanism received by a push rod and provided for the suture material, **characterized by** a device for forming an end-to-end anastomosis on two hollow organs, having
a) a carrier (9) designed as a hollow cylinder, with a sleeve (11) arranged coaxially with respect to the latter, wherein the longitudinal axis of the carrier (9) runs transversely with respect to the path of movement of the needle (2), and the jacket surface of the sleeve (11) is directed parallel to or at an acute angle to the plane of the needle plate (4) and serves as a contact surface for the edge areas of the two hollow organs to be connected, wherein
b) the carrier (9) and the sleeve (11) each have a longitudinal slot (13, 14) extending along at least part of the length thereof,
c) the sleeve (11) is rotatable relative to the carrier (9) by means of a drive, and the drive of the sleeve (11) is diverted from the drive of the suturing machine in such a way that the sleeve (11) executes an intermittent rotary movement, in accordance with the movement of the needle bar (1) and in phase with the suture formation.

7. Surgical suturing machine according to Claim 6, **characterized in that** the sleeve (11) has, at one of its end faces, a toothing (16) with which a driving pawl (17) mounted on the carrier (9) cooperates, said driving pawl (17) being connectable in drive terms to the drive of the needle bar (1) by a deformable transmission means (21).

8. Surgical suturing machine according to Claim 7, **characterized in that** the transmission means is a Bowden cable (21), one end of which is connected to the drive of the needle bar (1), and the other end of which acts on a lever (18) which accommodates the driving pawl (17) and which is mounted pivotably on the carrier (9).

9. Surgical suturing machine according to Claim 7, **characterized in that** the driving pawl (17) is arranged eccentrically with respect to the sleeve (11) and engages with a force fit on the inner face of the toothing (16).

10. Surgical suturing machine according to Claim 6, **characterized in that** the bracket (8) for the carrier (9) is formed by an angular carrying piece (26) whose first arm (27) is connectable to the housing of the suturing machine, and whose second arm (36) is connectable to the carrier (9), wherein the bracket (8) is movable, from the position securing the operating state of the carrier (9), to a position in which the carrier is directed substantially parallel to the longitudinal axis of the suturing machine.

11. Surgical suturing machine according to Claim 10, **characterized in that** the first arm (27) of the carrying piece (26) and the suturing machine are connectable to each other via a first hinge connection (28), and the second arm (36) of the carrying piece (26) and the carrier (9) are connectable to each other via a second hinge connection (35).

12. Surgical suturing machine according to Claim 11, **characterized in that** the first hinge connection (28) is connected releasably to the housing shaft (7) of the suturing machine.

13. Surgical suturing machine according to Claim 7, **characterized in that** the Bowden cable (21) is routed via a plurality of deflection sites (22, 23), of which at least one deflection site (22) is arranged on the carrier (9) and another deflection site is arranged on the first arm (27) of the carrying piece (26) or on the housing shaft (7).

14. Surgical suturing machine according to Claim 6, **characterized in that** the needle plate (4) has a contact surface for the first hollow organ and, at a vertical distance therefrom, an intermediate plate (41) that has a contact surface for the second hollow organ is provided.

## Revendications

1. Ensemble destiné à former une anastomose d'une extrémité à l'autre sur deux organes creux, apte à être monté sur une machine à coudre chirurgicale présentant
au moins une tringle à aiguille entraînée par moteur et présentant une aiguille de guidage de fil, un dispositif de saisie coopérant avec cette dernière, une plaque de piqûre et un support pour le produit cousu repris par une tringle de poussée,
**caractérisé par**
un support (9) configuré en cylindre creux et présentant une fente longitudinale (13) pour les parties de bord des deux organes creux à relier l'une à l'autre, le support pouvant être relié au moyen d'un soutien (8) à la tige (7) de boîtier de la machine à coudre de telle sorte que son axe longitudinal s'étende transversalement par rapport à la piste de déplacement de l'aiguille (2) de la machine à coudre, sa partie prévue pour la pose des deux organes creux étant orientée essentiellement parallèlement au plan de la surface de pose de la plaque de piqûre (4) de la machine à coudre et par
une douille (11) disposée coaxialement par rapport au support (9) et pouvant tourner par rapport à ce dernier, et dont la surface d'enveloppe sert de surface de pose pour les parties d'extrémité des deux organes creux, présentant une fente longitudinale (14) qui s'étend sur au moins une partie de sa longueur,
la douille (11) pouvant être mise en rotation par rapport au support (9) au moyen d'un entraînement et le déplacement de l'entraînement de la douille (11) étant dérivé de l'entraînement de la machine à coudre de telle sorte que la douille (11) exécute un déplacement de rotation intermittent en correspondance de phase avec la formation de la couture en fonction du déplacement de la tringle (1) à aiguille.

2. Ensemble selon la revendication 1, apte à être monté sur une machine à coudre chirurgicale, **caractérisé en ce que** la douille (11) peut être fermée au moyen d'un anneau de serrage (12) sur l'un de ses côtés frontaux et **en ce que** l'anneau de serrage (12) présente une fente longitudinale (15) qui correspond à la fente longitudinale (14) de la douille (11).

3. Ensemble selon la revendication 2, **caractérisé en ce que** la douille (11) présente à son extrémité non tournée vers l'anneau de serrage (12) une denture (16) avec laquelle coopère un cliquet d'entraînement (17) qui est monté sur le support (9) et qui peut être relié au moyen d'un moyen déformable de transfert (21) de manière à être entraîné par l'entraînement de la tringle (1) à aiguille.

4. Ensemble selon la revendication 1, **caractérisé en ce que** le soutien (8) du support (9) est formé d'une pièce porteuse (26) de forme coudée dont le premier bras (27) peut être relié par une première liaison articulée (28) à une pièce intermédiaire (29) fixée sur le boîtier de la machine à coudre chirurgicale et dont le deuxième bras (36) peut être relié au support (9) au moyen d'une deuxième liaison articulée (35), le soutien (8) pouvant être déplacé depuis la position qui assure la position de travail du support (9) jusque dans une position dans laquelle le support (9) est orienté essentiellement en parallèle à l'axe longitudinal de la machine à coudre.

5. Ensemble selon la revendication 4, **caractérisé en ce que** les deux liaisons articulées (28, 35) peuvent être bloquées.

6. Machine à coudre chirurgicale présentant au moins une tringle à aiguille entraînée par moteur et portant une aiguille de guidage de fil, un dispositif de saisie coopérant avec cette dernière, une plaque de piqûre et un support, repris par une tringle de poussée, pour le produit cousu, **caractérisée par** un ensemble de formation d'une anastomose d'extrémité à extrémité sur deux organes creux, la machine à coudre chirurgicale présentant
a.) un support (9) configuré comme cylindre creux et présentant une douille (11) disposée coaxialement par rapport au support, l'axe longitudinal du support (9) s'étendant transversalement par rapport à la piste de déplacement de l'aiguille (2) et la surface d'enveloppe de la douille (11) étant orientée parallèlement ou selon un angle aigu par rapport au plan de la plaque de piqûre (4) et servant de surface de pose pour les bordures des deux organes creux à relier,
b.) le support (9) et la douille (11) présentant chacun une fente longitudinale (13, 14) qui s'étend sur au moins une partie de leur longueur,
c.) la douille (11) pouvant être mise en rotation par rapport au support (9) au moyen d'un entraînement, l'entraînement de la douille (11) étant dérivé de l'entraînement de la machine à coudre de telle sorte que la douille (11) exécute un déplacement de rotation intermittent en correspondance de phase par rapport à la formation des piqûres en fonction du déplacement de la tringle (1) à aiguille.

7. Machine à coudre chirurgicale selon la revendication 6, **caractérisée en ce que** la douille (11) présente sur l'un de ses côtés frontaux une denture (16) avec laquelle coopère un cliquet d'entraînement (17) qui est monté sur le support (9) et qui peut être relié au moyen d'un moyen déformable de transfert (21) de manière à être entraîné par l'entraînement de la tringle (1) à aiguille.

8. Machine à coudre chirurgicale selon la revendication 7, **caractérisée en ce que** le moyen de transfert est un câble Bowden (21) dont une extrémité est reliée à l'entraînement de la tringle (1) à aiguille et dont l'autre extrémité engage un levier (18) reprenant le cliquet d'entraînement (17) et monté à pivotement sur le support (9).

9. Machine à coudre chirurgicale selon la revendication 7, **caractérisée en ce que** le cliquet d'entraînement (17) est disposé à l'extérieur de la douille (11) et engage le côté intérieur de la denture (16) en correspondance mécanique.

10. Machine à coudre chirurgicale selon la revendication 6, **caractérisée en ce que** le soutien (8) du support (9) est formé d'une pièce porteuse (26) de forme coudée dont le premier bras (27) peut être relié au fixée sur le boîtier de la machine à coudre chirurgicale et dont le deuxième bras (36) peut être relié au support (9), le soutien (8) pouvant être déplacé depuis la position qui assure la position de travail du support (9) jusque dans une position dans laquelle le support (9) est orienté essentiellement en parallèle à l'axe longitudinal de la machine à coudre.

11. Machine à coudre chirurgicale selon la revendication 10, **caractérisée en ce que** le premier bras (27) de la pièce de support (26) et la machine à coudre peuvent être reliées l'une à l'autre par une première liaison articulée (28) et le deuxième bras (36) de la pièce de support (26) et le support (9) peuvent être reliés l'un à l'autre par une deuxième liaison articulée (35).

12. Machine à coudre chirurgicale selon la revendication 11, **caractérisée en ce que** la première liaison articulée (28) est reliée de manière libérable à la tige (7) du boîtier de la machine à coudre.

13. Machine à coudre chirurgicale selon la revendication 7, **caractérisée en ce que** le câble Bowden (21) est guidé par plusieurs emplacements de renvoi (22, 23), dont au moins l'un (22) est disposé sur le support (9) et un autre sur le premier bras (27) de la pièce de support (26) ou sur la tige (7) du boîtier.

14. Machine à coudre chirurgicale selon la revendication 6, **caractérisée en ce que** la plaque de piqûre (4) présente une surface de pose pour le premier organe creux et **en ce qu'**à distance verticale par rapport à elle est prévue une plaque intermédiaire (41) qui présente une surface de pose pour le deuxième organe creux.
